# EUROPÄISCHE PATENTANMELDUNG

(11) **EP 4 733 985 A1**
(43) Veröffentlichungstag der Anmeldung: **29.04.2026**
(21) Anmeldenummer: 24208684.1
(22) Anmeldetag: 24.10.2024
(51) Int. Cl.: G06K 19/06, A61M 99/00, G16H 20/00

(54) **MEDIKATIONSBEHÄLTER UND INFUSIONSSYSTEM MIT EINEM SOLCHEN MEDIKATIONSBEHÄLTER**

(71) Anmelder: ConnCons GmbH, 01307 Dresden (DE)
(72) Erfinder: Gommel, Christoph, 01445 Radebeul (DE); Hampe, Jochen, 01277 Dresden (DE); Schurig, Carsten, 01445 Radebeul (DE)
(74) Vertreter: Witte, Weller und Partner Patentanwälte mbB Stuttgart

(57) **Zusammenfassung**

Bekannt sind Medikationsbehälter (10) insbesondere für die Anwendung im Rahmen einer Infusionstherapie. Häufig handelt es sich um formflexible Medikationsbeutel.

Ein solcher Medikationsbehälter (10) weist eine Wandung (20) auf, die einen Innenraum (12) umgibt. In diesem Innenraum wird die abzugebende Flüssigkeit gelagert. Der Medikationsbehälter (10) verfügt weiterhin über mindestens einen Fluidanschluss (30) zum Anschluss einer Fluidleitung (100) zum Zwecke der Entnahme von Flüssigkeit aus dem Medikationsbehälter.

Es wird vorgeschlagen, dass der Medikationsbehälter (10) eine erste Übertragungseinrichtung (40) zur Datenkommunikation mit einem auf einer Außenseite einer Wandung (20) angebrachten Informationsträger (70) aufweist.

## Beschreibung

### ANWENDUNGSGEBIET UND STAND DER TECHNIK

Die Erfindung betrifft Medikationsbehälter sowie ein Infusionssystem mit einem solchen Medikationsbehälter. Ein Medikationsbehälter gattungsgemäßer Art dient der Lagerung von medizinischen Flüssigkeiten vorderVerabreichungan den Patienten. Insbesondere kann ein solcher Medikationsbehälter als Medikationsbeutel ausgebildet sein, aber auch eine Version mit starren Wandungen ist möglich.

Unter medizinischen Flüssigkeiten im Sinne der Erfindung werden alle im Rahmen von medizinischen Therapien verwendeten Flüssigkeiten verstanden, beispielsweise auch Nährlösungen und Blutprodukte.

In zahlreichen Therapieverfahren, wie etwa der Krebstherapie, kommen maßgeschneiderte medizinische Flüssigkeiten zum Einsatz, die individuell auf die Bedürfnisse des Patienten abgestimmt werden. Die Verabreichung erfolgt aus Medikationsbehältern gattungsgemäßer Art. Die Medikationsbehälter mit solchen angepassten medizinischen Flüssigkeiten werden üblicherweise beschriftet, um Verwechslungen zu vermeiden.

Um zu gewährleisten, dass im Rahmen einer Therapie keine Fehler bei der Zuordnung vorkommen, also die Verwendung eines Medikationsbehälter, der nicht für den hiermit verbundenen Patienten vorgesehen ist, ist bereits vorgeschlagen worden, Medikationsbehälter mit einem Speicher zu versehen, so dass Informationen des Speichers von einem Steuergerät der Infusionssystems ausgelesen werden können.

### AUFGABE UND LÖSUNG

Aufgabe der Erfindung ist es, einen Medikationsbehälter bereitzustellen, der die Gefahr einer Verwechslung weiter verringert.

Erfindungsgemäß wird hierfür ein Medikationsbehälter vorgeschlagen, der in nachfolgend beschriebener Weise ausgestaltet ist. Ein Medikationsbehälter erfindungsgemäßer Art ist dabei üblicherweise technisch unaufwändig und als Einweg-Produkt ausgebildet.

Der erfindungsgemäße Medikationsbehälter weist in üblicher Weise eine Wandung auf, die einen Innenraum umgibt, in dem die zu verabreichende Flüssigkeit gelagert ist. Dies können beispielsweise sterile Flüssigkeiten, Medikamente oder Nährlösungen sein. Jegliche im Therapiekontext dem Patienten zugeführte Flüssigkeiten sind hier umfasst. Dies umfasst beispielsweise auch schlichtes Wasser, Lösungen, Blutbestandteile und Blut.

Die Wandungen sind üblicherweise primär aus biokompatiblem und chemisch stabilem Kunststoff hergestellt, um die Sterilität und Unversehrtheit der Flüssigkeiten zu gewährleisten. Insbesondere kommt hier PVC, Polyolefine, EVA oder Polyethylen in Frage. Auch können die Wandungen als metallische Wandungen oder Glaswandungen gestaltet sein. Die Wandungen können zusätzlich mit einem UV-Schutz versehen sein.

Insbesondere kann der Medikationsbehälter als Medikationsbeutel mit formflexibler Beutelwandung ausgebildet sein. Solche Beutel finden häufig bei Infusionstherapien Verwendung. Umfasst sind jedoch auch Medikationsbehälter mit starren Wandungen.

Der Medikationsbehälter verfügt über mindestens einen Fluidanschluss zum Anschluss einer Fluidleitung zum Zwecke der Entnahme von Flüssigkeit aus dem Medikationsbehälter und insbesondere zum Zwecke der Zuführung dieser Flüssigkeit zu einem Patienten über einen intravenösen Zugang.

Der Fluidanschluss des Medikationsbehälters kann beispielsweise in Art eines Luer-Konnektors ausgebildet sein.

Erfindungsgemäß wird vorgeschlagen, dass der Medikationsbehälter eine erste Übertragungseinrichtung zur Datenkommunikation mit einem auf einer Außenseite einer Wandung angebrachten Informationsträger aufweist. Diese erste Übertragungseinrichtung ist vorzugsweise an einerVorderwandung des Medikationsbehälters vorgesehen, insbesondere vorzugsweise in etwa in der Mitte derVorderwandung.

Während der bestimmungsgemäßen Verwendung des Medikationsbehälters ist ein solcher Informationsträger auf der Außenseite der Wandung angebracht, insbesondere dort aufgeklebt. Der Informationsträger verfügt über einen elektronischen Speicher, der an eine zweite Übertragungseinrichtung angeschlossen ist, die Teil dieses Informationsträgers ist.

Auf diese Weise können die erste und zweite Übertragungsvorrichtung genutzt werden, um vom Medikationsbehälters aus dem elektronischen Speicher des Informationsträgers auslesen zu können.

Die erste Übertragungseinrichtung und die zweite Übertragungseinrichtung können in verschiedener Weise ausgestaltet sein, um eine Datenkommunikation zwischen dem Medikationsbehälter und dem daran angebrachten Informationsträger zu gestatten. Eine einfache Möglichkeit besteht darin die ersten Übertragungseinrichtung in Form galvanisch leitfähiger Kontaktflächen auszugestalten, die an der Außenseite der Wandung offen liegen, gegebenenfalls nach Entfernung einer Schutzfolie. Korrespondierend hierzu sind auch auf der Rückseite des Informationsträgers galvanische Kontaktflächen vorgesehen, die nach Aufbringen des Informationsträgers an den Kontaktflächen des Medikationsbeutels anliegen.

Die Übertragungsvorrichtung kann alternativ dazu auch als kapazitive oder induktive Übertragungsvorrichtung ausgebildet sein.

Eine kapazitive Übertragungsvorrichtung, die in die Wandung eingebracht ist, umfasst dünne leitfähige Kondensatorflächen, die vorzugsweise nach innen und außen durch Folien umschlossen sind und dadurch elektrisch nach außen isoliert sind. Die leitfähigen Kondensatorflächen wirken dabei als Elektroden, die elektrische Felder erzeugen und empfangen können. Korrespondierend hierzu sind auch im Informationsträger Kondensatorflächen vorgesehen.

Eine induktive Übertragungsvorrichtung weist eine Übertragungsspule auf, die in die Wandung integriert ist. Es handelt sich vorzugsweise um eine planare und insbesondere vorzugsweise flexible Übertragungsspule, die außenseitig und innenseitig durch Folienlagen der Wandung umschlossen ist. Die Übertragungsspule besteht aus einer oder mehreren Windungen leitfähigen Materials, typischerweise aus Kupfer oder Aluminium. Die überdeckende Folienlage kann bspw. an der Wandung auflaminiert sein, durch ein Spritzgusseinlegeteil (In-mold labelling) oder durch Ultraschall- oder Laserverschweißung hier angebracht sein.

Die Übertragungsspule kann insbesondere in Art einer durch Druck- oder Beschichtungstechnik aufgebrachten Lage ausgebildet sein oder aus einem flächigen Material herausgeschnitten sein, insbesondere mittels Laserschnitts, und dann in die Wandung integriert worden sein. Auch die Erzeugung mittels Beschichtung oder Druck ist möglich.

Die Übertragungsspule kann Teil eines NFC-Tags sein, welches einschließlich eines NFC-Chips auf bzw. in der Wandung des Medikationsbehälters eingebracht ist.

Vorzugsweise ist eine Trägerfolie vorgesehen, die auf einer Wandung des Medikationsbehälters angebracht ist und an der die Übertragungsspule oder die Kondensatorflächen der ersten Übertragungsvorrichtung angebracht ist. Diese Trägerfolie wird gemeinsam mit der Übertragungsspule oder den Kondensatorflächen an einer Vorderwandung der Medikationsbehälters angebracht. Der Informationsträger wird außenseitig auf dieser Folie angebracht, insbesondere aufgeklebt.

Im Falle eines Medikationsbeutels kann die genannte Trägerfolie eine Größe aufweisen, die der Größe der Vorderwandung entspricht, auf die sie aufgebracht wird, so dass sie eine die gesamte Vorderwandung abdeckende Folie bildet.

Vorzugsweise ist die erste Übertragungseinrichtung über Signalleitungen mit einem Datenkonnektor verbunden, so dass die erste Übertragungseinrichtung über diesen Datenkonnektor mit einer externen Steuereinrichtung verbindbar ist. Dieser Datenkonnektor am Medikationsbehälter gestattet es, den Medikationsbehälter an eine Datenleitung anzuschließen, die zu einer externen Steuereinrichtung führt, so dass diese Steuereinrichtung über den Datenkonnektor sowie über die erste und zweite Übertragungseinrichtung mit dem Speicher des Informationsträgers verbunden ist und diesen auslesen kann.

Der Datenkonnektor kann in nachfolgend noch beschriebener Weise insbesondere in den Fluidanschluss integriert sein.

Die genannte Trägerfolie, die mindestens die erste Übertragungsvorrichtung trägt, kann zusammen mit dem Fluidanschluss mit integriertem Datenkonnektor eine Vormontageeinheit bilden, die bereits vor Anbringung der Trägerfolie an der Wandung eine zusammengefügte Teileinheit bildet. Dies kann die Montage erleichtern, da alle elektrischen Leitungen zwischen der ersten Übertragungsvorrichtung und dem Datenkonnektor bereits Teil der genannten Vormontageeinheit sind und damit die galvanische Konnektierung zwischen Datenkonnektor und erster Übertragungseinrichtung im Zuge der Anbringung der Trägerfolie entfällt.

Grundsätzlich ist es möglich, dass der Medikationsbehälter selbst eine Steuereinrichtung in Form eines integrierten Schaltkreises enthält, der die Datenkommunikation über die erste Übertragungseinrichtung und/oder über den Datenkonnektor steuert. Bevorzugt ist jedoch eine Gestaltung, bei der die Steuereinrichtung, die die Datenkommunikation über die erste Übertragungseinrichtung steuert, nicht Teil des Medikationsbehälters ist, sondern extern vorgesehen ist, beispielsweise integriert in eine Pumpe des Infusionssystems oder hieran angeschlossen. Dieses externe Steuergerät ist über den Datenkonnektor mit dem Medikationsbehälter und damit mit der ersten Übertragungseinrichtung verbunden.

Die Signalleitungen des Medikationsbehälters sind vorzugsweise einheitlich zur Übertragungsspule, den Kontaktflächen oder den Kondensatorflächen ausgebildet, also zu diesen einstückig ausgebildet. Insbesondere können die Signalleitungen gemeinsam mit der als Übertragungsspule oder Kondensatorfläche ausgebildeten ersten Übertragungseinrichtung aus einer gemeinsamen Metallfolie hergestellt werden.

Der Datenkonnektor ist vorzugsweise ortsfest zum mindestens einen Fluidanschluss ausgebildet, so dass durch Anschließen eines Anschlussstücks einer Fluidleitung am Fluidanschluss gleichzeitig ein am Anschlussstück vorgesehener zweiter Datenkonnektor mit dem Datenkonnektor des Medikationsbeutels verbindbar ist.

Insbesondere kann der Datenkonnektor in den Fluidanschluss integriert sein. Durch Anschließen des Anschlussstücks am Fluidanschluss wird bei einer solchen Gestaltung auch die Datenverbindung geschaffen. In Hinblick auf die Gestaltung des Fluidanschlusses wird auf das Dokument WO 2021/023639 A1 und dessen Parallelveröffentlichungen verwiesen, deren Offenbarung in Hinblick auf die Gestaltungen von Anschlussstücken als Fluidanschluss durch explizite Bezugnahme zur Offenbarung der vorliegenden Erfindung gemacht wird.

Der Datenkonnektor kann insbesondere in Form von galvanischen Kontaktflächen am Fluidanschluss vorgesehen sein. Im Falle einer Übertragungsspule zur Datenübertragung am Fluidanschluss ist diese Übertragungsspule vorzugsweise die Fluidleitung des Fluidanschlusses umgebend ausgebildet.

Der Informationsträger, der insbesondere als Aufkleber ausgestaltet ist und mit der zweiten Übertragungsvorrichtung ausgestattet ist, beinhaltet vorzugsweise Informationen nicht nur im genannten elektronischen Speicher, sondern ist auch mit einer visuell erfassbaren Beschriftung versehen, die vorzugsweise hier aufgedruckt ist.

Dabei ist insbesondere eine Verwendung vorgesehen, bei der die visuell erfassbare Beschriftung und Daten im Speicher zumindest teilweise übereinstimmen.

Eine solche Gestaltung, bei der sowohl digitale Daten des Informationsträgers als auch dessen Beschriftung übereinstimmen, bietet einen erheblichen Vorteil, so dass es nicht zu Abweichungen kommen kann, wenn die Beschriftung des Informationsträgers und das Beschreiben von dessen Speicher in einem gemeinsamen Prozess stattfindet. Es besteht somit Sicherheit, dass nicht durch eine Verwechslung die Beschriftung und die Informationen im Speicher voneinander abweichen.

Der Informationsträger weist vorzugsweise einen mehrlagigen Aufbau auf, insbesondere mit einer nach innen weisenden Trägerfolie, einer Übertragungsspule als zweite Übertragungsvorrichtung und mit einer nach außen weisenden Druckfläche zur Aufnahme der Beschriftung. Im vorbereiteten Zustand ist der Medikationsbehälter mit dem Informationsträger versehen, wobei dieser bedruckt ist und sein elektronischer Speicher mit Informationen korrespondierend zu den gedruckten Informationen beschrieben ist.

Der Informationsträger kann zusätzlich auch weitere elektronische Komponenten aufweisen. Insbesondere kommen hier Sensoren in Betracht, die die Temperatur erfassen und über die erste und die zweite Übertragungseinrichtung von außen auslesbar zur Verfügung stellen.

Die Erfindung betrifft neben dem Medikationsbehälter als solchem auch ein Infusionssystem, in welchen ein Medikationsbehälter der vorbeschriebenen Art verwendet wird. In Hinblick auf den Aufbau eines solchen Infusionssystems wird auf das Dokument WO 2021/023639 A1 und dessen Parallelveröffentlichungen verwiesen, deren Offenbarung in Hinblick auf die Gestaltung des Infusionssystems durch explizite Bezugnahme zur Offenbarung der vorliegenden Erfindung gemacht wird.

Ein erfindungsgemäßes Infusionssystem umfasst eine Steuereinrichtung, insbesondere eine in eine Pumpeinrichtung integrierte Steuereinrichtung oder eine an der Pumpeinrichtung über eine Datenleitung angeschlossene Steuereinrichtung, sowie mindestens einen Medikationsbehälter, der über eine Fluidleitung angeschlossen ist.

Dabei ist der Medikationsbehälter in der oben beschriebenen Art ausgebildet.

Die genannte Steuereinrichtung ist über eine Datenleitung mit dem Medikationsbehälter verbindbar, wobei die Datenleitung vorzugsweise in einem Datenkonnektor endet, der an einem korrespondierenden Datenkonnektor des Medikationsbehälters angeschlossen werden kann. Die Datenleitung ist vorzugsweise in die Fluidleitung integriert. Die Datenkonnektoren auf Seiten des Medikationsbehälters und auf Seiten der Fluidleitung sind vorzugsweise jeweils in Fluidanschlüsse integriert, insbesondere in zusammensteckbare Luer-Konnektoren.

Die Steuereinrichtung ist dafür ausgebildet, Daten auf dem am Medikationsbehälter angebrachten Informationsträger über die erste Übertragungseinrichtung des Medikationsbehälter und die zweite Übertragungseinrichtung des Informationsträgers auszulesen.

Diese Daten können dann zur Validierung anhand eines vorliegenden Medikationsplans herangezogen werden. Auch möglich ist es, dass der Medikationsplan selbst im Speicher des Informationsträgers abgelegt ist und von der Steuereinrichtung ausgelesen wird.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend an-hand der Figuren erläutert sind.
Fig. 1 zeigt ein erfindungsgemäßes Infusionssystem mit erfindungsgemäßen Medikationsbehältern.
Fig. 2 zeigt einen erfindungsgemäßen Medikationsbehälter vor Anbringung eines Informationsträgers.
Fig. 3 zeigt den Informationsträger.
Fig. 4 zeigt den Medikationsbehälter mit angebrachtem Informationsbehälter.
Fig. 5 zeigt den Fluidanschluss des Medikationsbehälters und verdeutlicht die Datenübertragung von Medikationsbehälterzu einer externen Steuereinrichtung.

### DETAILLIERTE BESCHREIBUNG DER AUSFÜHRUNGSBEISPIELE

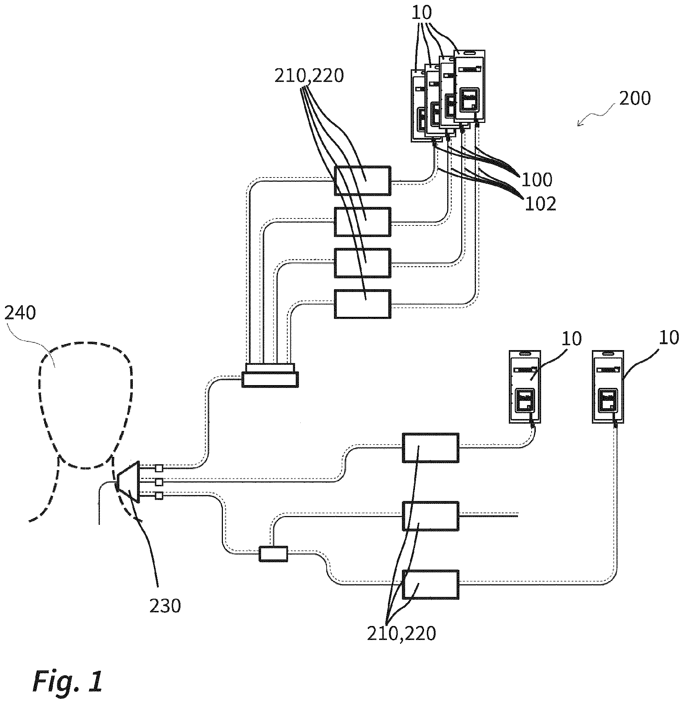

Figur 1 zeigt die schematische Darstellung eines erfindungsgemäßen Infusionssystems 200. Das Infusionssystem 200 weist eine Anzahl von Medikationsbehältern 10 in Form von formflexiblen Beuteln auf. Die Medikationsbehältern 10 sind über Fluidleitungen 100 mit Infusionspumpen 210 verbunden.

Die Infusionspumpen 210 sind dafür ausgebildet, die Flüssigkeiten aus den Medikationsbehältern 10 gemäß einem Medikationsplan zu einem zentralen Venenkatheter 230 eines Patienten 240 zu fördern.

Die Infusionspumpen 210 weisen jeweils eine Steuereinrichtung 220 auf, die die Infusionspumpe steuert. Diese Steuerung der Infusionspumpen 210 bezieht dabei bei einem erfindungsgemäßen Infusionssystem 200 auch Daten mit ein, die von den Medikationsbehältern 10 stammt.

Zu diesem Zweck sind die Medikationsbehälter 10 jeweils mit einem Speicher versehen, wie im Weiteren noch erläutert werden wird.

Die Datenübertragung zwischen den Steuereinrichtungen 220 und den Medikationsbehältern 10 erfolgt über Datenleitungen 102, die in die Fluidleitungen 100 integriert sind. Diese Datenleitungen 102 sind mit einer Datenleitung an den Infusionspumpen 210 und hierüber mit den Steuereinrichtungen 220 verbunden.

Auf der Seite der Medikationsbehälter 10 sind die Datenleitungen 102 über Anschlusstücke 30, 110 mit den Medikationsbehältern 10 verbunden, die im Weiteren noch detailliert erläutert werden.

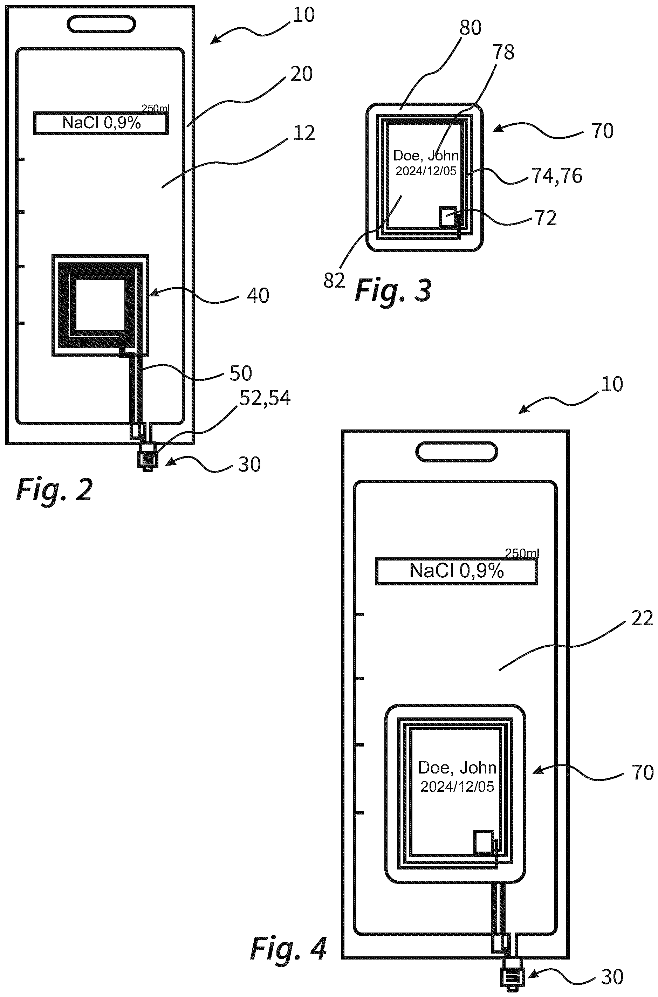

Fig. 2 und 4 zeigen einen Medikationsbehälter 10 in Form eines formflexiblen Medikationsbeutels sowie in Fig. 3 einen hierauf angebrachten Informationsträger 70.

Der Medikationsbehälter 10, der in Fig. 2 dargestellt ist, weist noch keinen solchen Informationsträger 70 auf. Wie aus der Figur 2 zu entnehmen ist, weist der Medikationsbehälter 10 Wandungen 20, nämlich eine Vorderwandung 22 und eine hintere Wandung, auf, die einen Innenraum 12 umgeben, in dem die medizinische Flüssigkeit gelagert ist. Am unteren Ende weist der Medikationsbehälter 10 einen Fluidanschluss 30 zum Anschluss einer Fluidleitung 100 auf.

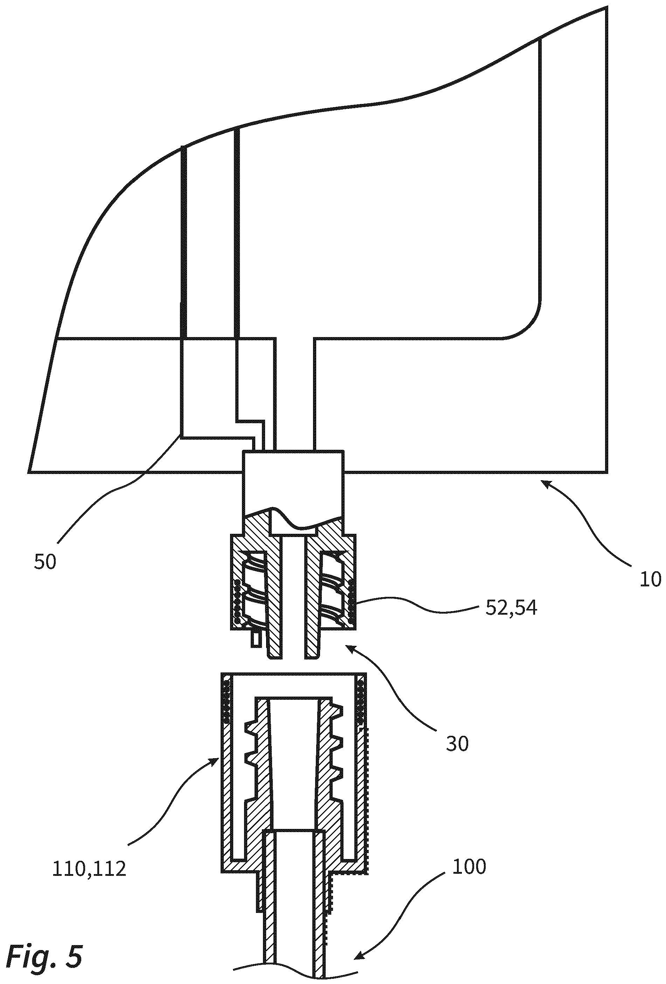

Weiterhin weist der Medikationsbehälter 10 an der Vorderwandung 22 eine erste Übertragungseinrichtung40 in Form einer Übertragungsspule auf. Diese Übertragungsspule ist über Signalleitungen 50 mit einem Datenkonnektor 52 verbunden, der ebenfalls über eine Übertragungsspule 54 verfügt und der beim vorliegenden Ausführungsbeispiel in den Fluidanschluss 30 integriert ist. Dies wird im Weiteren anhand der Fig. 5 näher erläutert.

Der in Fig. 3 dargestellte Informationsträger 70 ist dafür bestimmt, auf der Vorderwandung 22 des Medikationsbehälters 10 angebracht zu werden. Der Informationsträger 70 verfügt über einen elektronischen Speicher 72, der an eine Übertragungsspule 76 einer zweiten Übertragungseinrichtung 74 angeschlossen ist. Es kann sich bei dem Speicher 72 und der Übertragungsspule 76 um Elemente eines handelsüblichen NFC-Tags handeln. Neben dem elektronischen Speicher 72 verfügt der Informationsträger über eine Druckfläche 82, die zur Aufnahme einer Beschriftung vorgesehen ist oder im vorliegenden Fall bereits mit einer Beschriftung 78 versehen ist. Beim vorliegenden Beispiel besteht die Beschriftung aus dem Namen des Patienten sowie aus einem Datum, welches die Haltbarkeit des Inhalts des Medikationsbehälters 10 angibt. Andere Daten, die im Speicher abgelegt sein können, sind eine ID, der in einem externen Zentralspeicher Daten zugeordnet sind. Auch kann im Speicher ein Nachweis über die Echtheit des Medikaments und/oder eine digitale Signatur des Erstellers hinterlegt sein.

Die Besonderheit des Informationsträgers 70 liegt unter anderem darin, dass er die gleichen Informationen, vorliegend beispielsweise das Haltbarkeitsdatum, in elektronischer Form im Speicher 72 und aufgedruckt in Form der Beschriftung 78 enthält. Diese Informationen werden vorzugsweise in einem einheitlichen Prozess aufgebracht. Es kann beispielsweise ein Drucker mit integrierter NFC-Übertragungseinrichtung vorgesehen sein, der in einem einheitlichen Prozess die Beschriftung aufbringt und die gleichen Daten im Speicher 72 ablegt.

Der Informationsträger 70 kann weitere elektronische Komponenten aufweisen. So kann er insbesondere auch Sensoren umfassen, beispielsweise einen Temperatursensor, um anhand dessen zu überwachen, ob die Flüssigkeit stets auf Solltemperatur gehalten ist. Sofern die Erfassung von Daten mittels dieser Sensoren auch möglich sein soll, wenn der Medikationsbehälter 10 nicht an die Datenleitung 102 angeschlossen ist und nicht mit Strom versorgt ist, so kann zu diesem Zwecke eine Batterie und/oder eine Auswertungselektronik auf dem Informationsträger oder als Teil des Medikationsbehälters vorgesehen werden.

Fig. 4 zeigt den Medikationsbehälters 10 mit aufgebrachtem Informationsträger 70. Der Informationsträger 70 verfügt zum Zwecke der Aufbringung über eine Trägerfolie 80, an der rückseitig eine Klebefläche vorgesehen sein kann. Mittels dieser Klebefläche ist der Informationsträger 70 auf derVorderwandung22 des Medikationsbehälters 10 aufgebracht worden, wobei die erste Übertragungseinrichtung 40 und deren Übertragungsspule 42 an der Vorderwandung 22 des Medikationsbehälters 10 von dem Informationsträger 70 sowie insbesondere von dessen zweiter Übertragungseinrichtung 74 und deren Übertragungsspule 76 überdeckt ist.

Durch diese Art der Anbringung lässt sich der Speicher 72 vom Datenkonnektor 52 aus auslesen. Auch Sensordaten können über den Datenkonnektor52 ggf. ausgelesen werden.

Fig. 5 verdeutlicht, wie die Datenübertragung von der Fluidleitung 100 auf den Medikationsbehälters 10 erfolgt. Am Ende der Fluidleitung 100 ist ein Anschlussstück 110 vorgesehen, beispielsweise in Art eines weiblichen Luer-Konnektors. Am Medikationsbehälters 10 ist ebenfalls ein Anschlussstück 30 vorgesehen, welches den Fluidanschluss 30 bildet und welches beispielsweise in Art eines männlichen Luer-Konnektors ausgebildet sein kann.

Beide Anschlussstücke 30, 110 sind mit integriertem Datenkonnektor 52, 112 ausgerüstet. Diese Datenkonnektoren 52, 112 sind jeweils mit einer Übertragungsspule 54, 114 ausgerüstet, so dass eine induktive Datenübertragung hier ermöglicht wird. Auf der Seite des Medikationsbehälters 10 ist diese Übertragungsspule 54 mit den Signalleitungen 50 verbunden, die in bereits beschriebener Weise am gegenüberliegenden Ende mit der Übertragungsspule 42 verbunden sind. Auf der Seite der Fluidleitung 100 ist die Übertragungsspule 114 mit der Datenleitung 102 und somit mittelbar mit der zugehörigen Steuereinrichtungen 220 verbunden.

Auf diese Weise ist den Steuereinrichtungen 220 möglich, über die Übertragungsspulen 54, 114 sowie über die Übertragungsspule 42, 76 auf den Speicher 72 zuzugreifen und die darin enthaltenen Informationen elektronisch auszulesen. Die Steuereinrichtungen 220 können auf diesem Wege beispielsweise ermitteln, ob der Medikationsbehälter 10 tatsächlich für den am Venenkatheter 230 angeschlossenen Patienten 240 vorgesehen ist. Ist dies nicht der Fall, kann eine Flüssigkeitsförderung mittels der Pumpeinrichtung 210 unterbunden werden.

Gleichzeitig ist durch die Beschriftung 78 und deren oben beschriebenen Erstellung in einem einheitlichen Prozess mit der Hinterlegung von Daten im Speicher 72 auch gewährleistet, dass die Daten des Informationsträgers 70 konsistent sind. Es kann also nicht vorkommen, dass zwar die Beschriftung 78 den Eindruck vermittelt, der Medikationsbehälter sei für den Patienten 240 vorgesehen, die Daten im Speicher 72 dem jedoch widersprechen.

## Patentansprüche

1. Medikationsbehälter (10), insbesondere für die Anwendung im Rahmen einer Infusionstherapie, mit den folgenden Merkmalen:
a. der Medikationsbehälter (10) weist eine Wandung (20) auf, die einen Innenraum (12) umgibt, und
b. der Medikationsbehälter (10) verfügt über mindestens einen Fluidanschluss (30) zum Anschluss einer Fluidleitung (100) zum Zwecke der Entnahme von Flüssigkeit aus dem Medikationsbehälter,
**gekennzeichnet durch** das folgende weitere Merkmal:
c. der Medikationsbehälter (10) weist eine erste Übertragungseinrichtung (40) zur Datenkommunikation mit einem auf einer Außenseite einer Wandung (20) angebrachten Informationsträger (70) auf.

2. Medikationsbehälter (10) nach Anspruch 1 mit den folgenden weiteren Merkmalen:
a. der Medikationsbehälter (10) ist als Medikationsbeutel ausgebildet, und
b. die Wandung (20) ist als formflexible Beutelwandung ausgebildet.

3. Medikationsbehälter (10) nach Anspruch 1 oder 2 mit den folgenden weiteren Merkmalen:
a. die erste Übertragungseinrichtung (40) umfasst galvanisch leitfähige Kontaktflächen an der Außenseite der Wandung, oder
b. die erste Übertragungseinrichtung (40) ist als kapazitive Übertragungseinrichtung mit eingebetteten Kondensatorflächen ausgebildet.

4. Medikationsbehälter nach Anspruch 1 oder 2 mit dem folgenden weiteren Merkmal:
a. die erste Übertragungseinrichtung (40) weist eine Übertragungsspule (42) auf, vorzugsweise mit den folgenden weiteren Merkmalen:
b. die Übertragungsspule (42) ist als planare Übertragungsspule ausgebildet, die sich in der Ebene einer Wandung des Medikationsbehälters befindet, und/oder
c. die Übertragungsspule (42) ist aus metallischem Material gebildet, welches vorzugsweise durch Druck und Beschichtung oder durch Laserschnitt in Übertragungsspulenform gebracht wurde, und/oder

5. Medikationsbehälter nach Anspruch 4 mit dem folgenden weiteren Merkmal:
a. es ist eine Trägerfolie vorgesehen, die auf einer Wandung des Medikationsbehälters angebracht ist und an der die Übertragungsspule oderdie Kondensatorflächen angebracht ist,
vorzugsweise mit einem der folgenden weiteren Merkmale:
b. die Trägerfolie weist eine Größe auf, die der Größe der Wandung entspricht, auf der sie angebracht ist, und/oder
c. die Trägerfolie bildet zusammen mit dem Fluidanschluss eine Vormontageeinheit, die bereits vor Anbringung der Trägerfolie an der Wandung eine zusammengefügte Teileinheit bildet.

6. Medikationsbehälter (10) nach einem der vorstehenden Ansprüche mit dem folgenden weiteren Merkmal:
a. die erste Übertragungseinrichtung (40) ist über Signalleitungen (50) mit einem Datenkonnektor (52) verbunden, über den die erste Übertragungseinrichtung (40) mit einer Steuereinrichtung (210) verbindbar ist,
vorzugsweise mit dem folgenden weiteren Merkmal:
b. die Steuereinrichtung (220) ist nicht Teil des Medikationsbehälter (10), und/oder
c. mindestens eine Signalleitung (50) ist einheitlich zur als Übertragungsspule (42) ausgebildeten Übertragungseinrichtung (40) ausgebildet, insbesondere in Form eines aus einer Metallfolie hergestellten Verbundes aus Übertragungsspulenwindungen und Signalleitung (50).

7. Medikationsbehälter (10) nach Anspruch 6 mit dem folgenden weiteren Merkmal:
a. der Datenkonnektor (52) ist ortsfest zum mindestens einen Fluidanschluss (30) ausgebildet, so dass durch Anschließen eines Anschlussstücks (110) einer Fluidleitung (100) am Fluidanschluss (30) gleichzeitig ein am Anschlussstück (110) vorgesehener zweiter Datenkonnektor (112) mit dem Datenkonnektor (52) des Medikationsbeutels (10) verbindbar ist.

8. Medikationsbehälter (10) nach Anspruch 7 mit dem folgenden weiteren Merkmal:
a. der Datenkonnektor (52) ist in den Fluidanschluss (30) integriert,
vorzugsweise mit dem folgenden weiteren Merkmal:
b. der Datenkonnektor (52) weist galvanische Kontaktflächen und/oder eine Übertragungsspule (54) auf.

9. Medikationsbehälter (10) nach einem der vorstehenden Ansprüche mit den folgenden weiteren Merkmalen:
a. im Bereich der ersten Übertragungseinrichtung (40) ist ein Informationsträger (70) auf einer Wandung (20) des Medikationsbehälters (10) angebracht, und
b. der Informationsträger (70) weist einen elektronischen Speicher (72) und eine zweite Übertragungseinrichtung (74), vorzugsweise mit einer Übertragungsspule (76) zur induktiven Datenübertragung auf, so dass über die erste und die zweite Übertragungseinrichtung (40, 74) der Inhalt des elektronischen Speichers (72) auslesbar ist.

10. Medikationsbehälter (10) nach einem der vorstehenden Ansprüche mit dem folgenden weiteren Merkmal:
a. der Informationsträger (70) weist eine visuell erfassbare Beschriftung (78) auf, vorzugsweise mit mindestens einem der folgenden zusätzlichen Merkmale:
b. die visuell erfassbare Beschriftung (78) und Daten im Speicher (72) stimmen zumindest teilweise überein, und/oder
c. der Informationsträger (70) ist als Aufkleber ausgebildet, und/oder
d. der Informationsträger (70) weist einen mehrlagigen Aufbau auf, insbesondere mit einer nach innen weisenden Trägerfolie (80), der Übertragungsspule (76) und einer nach außen weisenden Druckfläche (82).

11. Medikationsbehälter (10) nach Anspruch 9 oder 10 mit den folgenden weiteren Merkmalen:
a. der Informationsträger (70) weist mindestens einen Sensor auf, und
b. der Sensor ist über die erste und die zweite Übertragungseinrichtung (40, 74) auslesbar.

12. Medikationsbehälter (10) nach einem der vorstehenden Ansprüche mit dem folgenden weiteren Merkmal:
a. die erste Übertragungseinrichtung (40) ist an einer Vorderwandung (22) des Medikationsbehälters (10) vorgesehen, vorzugsweise in der Mitte der Vorderwandung (22).

13. Infusionssystem (200) mit den folgenden Merkmalen:
a. das Infusionssystem (200) umfasst eine Steuereinrichtung (220), insbesondere eine in eine Pumpeinrichtung (210) integrierte Steuereinrichtung (220) oder eine an der Pumpeinrichtung (210) über eine Datenleitung angeschlossene Steuereinrichtung (220), und
b. das Infusionssystem (200) umfasst mindestens einen Medikationsbehälter (10), der über eine Fluidleitung (100) angeschlossen ist,
**gekennzeichnet durch** die folgenden zusätzlichen Merkmale,
c. der Medikationsbehälter (10) ist nach einem der vorstehenden Ansprüche ausgebildet, und
d. die Steuereinrichtung (210) ist über eine Datenleitung mit dem Medikationsbehälter (10) verbunden, und
e. die Steuereinrichtung (210) ist dafür ausgebildet, Daten auf einem am Medikationsbeutel (10) angebrachten Informationsträger (70) überdie erste Übertragungseinrichtung (40) des Medikationsbeutels (10) auszulesen.
